**Europäisches Patentamt**

**European Patent Office**

**Office européen des brevets**

(11) Publication number: **0 373 810**
**A1**

## EUROPEAN PATENT APPLICATION

(21) Application number: **89312638.3**

(22) Date of filing: **04.12.89**

(51) Int. Cl.5: **C07C 271/04, C07C 269/00**

(30) Priority: **10.12.88 GB 8828915**

(43) Date of publication of application:
**20.06.90 Bulletin 90/25**

(84) Designated Contracting States:
**AT BE CH DE ES FR GB GR IT LI LU NL SE**

(71) Applicant: **SCHERING AGROCHEMICALS LIMITED**

**Hauxton Cambridge CB2 5HU(GB)**

(72) Inventor: **Taylor, Robert William**
**Norris Close 21 King Street**
**Rampton Cambridge(GB)**
Inventor: **Pollard, Michael David**
**137 Greenfields**
**Earith Cambridge(GB)**

(74) Representative: **Waldman, Ralph David et al**
**Schering Agrochemicals Limited Industrial Property Department Chesterford Park Research Station**
**Saffron Walden Essex CB10 1XL(GB)**

(54) **Preparation of carbamoyl halides.**

(57) Compounds of formula I
RNHCOCl      (I)
where R is an optionally substituted alkyl or aryl group, are obtained by chlorinating a compound of formula II
RNHCHO      (II)
in the absence of a solvent.

EP 0 373 810 A1

## Preparation of carbamoyl halides

This invention relates to a new process for the preparation of carbamoyl chlorides.

It is known that methylcarbamoyl chloride can be used for the preparation of carbamate esters, especially those carbamates which have pesticidal properties, (see eg GB 1,594,422).

In GB 2,101,130, there is described the preparation of alkylcarbamoyl halides by halogenating the corresponding formamide. The compounds were not isolated but reacted in situ with a salt of a benzoic acid sulphimide. The specification states that the reaction is preferably carried out in a solvent, generally an aprotic solvent. The only halogenating agent which is exemplified is sulphuryl chloride, which is used in the presence of chloroform. Elemental halogen is also mentioned as a possible halogenating agent. We have found however that when attempting to chlorinate N-methylformamide with chlorine in the presence of chloroform as solvent, little or no conversion to methylcarbamoyl chloride occurs. Surprisingly however, when the solvent was omitted and the reaction run continuously, very high yields of methylcarbamoyl chloride were achieved. We also found that if sulphuryl chloride is used as the chlorinating agent in the absence of solvents, in both batch and continuous reactions, increased yields are achieved and the reaction time is also reduced.

Thus according to the invention there is provided a process for the preparation of a compound of formula I

RNHCOCl     (I)

where R is an optionally substituted alkyl or aryl group, which comprises chlorinating a compound of formula II

RNHCHO     (II)

in the absence of a solvent.

R is preferably a $C_{1-4}$-alkyl group, especially methyl.

The chlorinating agent is generally elemental chlorine. However, other chlorine generating agents, such as sulphuryl chloride, can also be used. The reaction is generally carried out at such a temperature that the reaction mixture remains liquid. The temperature is generally between 40 to 70°C, preferably 60 to 65°C, especially when the compound of formula II is N-methylformamide. As the the reaction temperature is increased, so can the residence time (ie the ratio of reactor volume to feed rate of N-methylformamide) be reduced.

The molar ratio of chlorinating agent to formamide is preferably from 1.1:1 to 1:1. Preferably the reaction is carried out as a continuous process, especially when using chlorine. In general the reaction is carried out without any other additives.

The invention is illustrated in the following examples.

## Example 1

N-methylformamide (118.1 g) was added over 2 hours with cooling and stirring to sulphuryl chloride (300 g). The mixture was then stirred at 42°C for one hour and evaporated under reduced pressure to give 189.1 g of a white solid which on analysis was shown to comprise 80% methylcarbamoyl chloride, representing a yield of about the same figure.

## Example 2

A reaction vessel consisting of a jacketed round flask having an overflow side arm, addition tubes for N-methylformamide and sulphuryl chloride and a vent pipe attached to a recirculatory caustic soda scrubber to remove liberated hydrogen chloride, was charged with molten methylcarbamoyl chloride. The reactor was maintained at 40-45°C whilst sulphuryl chloride and N-methylformamide were added at a molar ratio of 1-1.1:1 at a feed rate of 0.85 g N-methylformamide per minute. The reaction was allowed to run continuously, whereby methylcarbamoyl chloride overflowed into a collecting vessel where it solidified after degassing (by maintaining a slightly reduced pressure in this vessel). The product was shown to be at least 88% pure methylcarbamoyl chloride.

## Example 3

Example 2 was repeated in which addition of sulphuryl chloride was replaced by addition of chlorine in a similar molar ratio via a sparge pipe. Methylcarbamoyl chloride having a purity of at least 90% was obtained.

## Example 4

Example 3 was repeated using a temperature of 60° and a consequent increase in feed rate of 1.94 g N-methylformamide per minute. Methylcarbamoyl chloride having a purity of at least 90% was obtained.

## Comparative Example 1

Chlorine gas (5 g) is added via a sparge pipe, over 45 minutes, to a stirred solution of N-methylformamide (4 g) in chloroform (60 ml) whilst maintaining the temperature at 2 to 8°C. The reaction vessel contains a vent pipe attached to a recirculatory caustic soda scrubber to remove liberated hydrogen chloride. The mixture was stirred overnight at room temperature under an atmosphere of dry nitrogen to exclude moisture, which might cause decomposition of methylcarbamoyl chloride. Analysis showed that less than 1% methylcarbamoyl chloride was formed.

Comparative Example 2

Comparative Example 1 was repeated in a similar manner but adding sulphuryl chloride (11.5 g) to N-methylformamide (5 g) in chloroform (80 ml). Analysis showed that less than 50% methylcarbamoyl chloride was formed.

**Claims**

1. A process for the preparation of a compound of formula I
RNHCOCl      (I)
where R is an optionally substituted alkyl or aryl group, which comprises chlorinating a compound of formula II
RNHCHO      (II)
in the absence of a solvent.

2. A process according to claim 1, in which R is a $C_{1-4}$-alkyl group.

3. A process according to claim 2, in which R is methyl.

4. A process according to any one of claims 1 to 3, in which the chlorinating agent is elemental chlorine.

5. A process according to any one of the preceding claims, which is carried out at a temperature of 40 to 70°C.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,Y | CHEMICAL ABSTRACTS, vol. 99, no. 9, 29th August 1983, page 630, abstract no. 70727k, Columbus, Ohio, US; & HU-A-24 125 (CHINOIN GYOGYAZER ES VEGYESZETI TERMEKEK GYARA RT.) 28-12-1982 * Abstract * | 1-5 | C 07 C 271/04 C 07 C 269/00 |
| Y | DE-A-1 094 737 (BAYER) * Examples 1-4 * | 4 | |
| A | J. AMER. CHEM. SOC., vol. 44, 1922; R.S. BLY et al.: "The preparation of phenylimido-phosgene, and the chlorination of formanilide", pages 2896-2903 | 1 | |
| D,Y | FR-A-2 507 599 (CHINOIN) * Examples 1-5 * & GB-A-2 101 130 | 1-5 | |
| A | GB-A-1 027 349 (PROGIL) * Entire document * | 1 | TECHNICAL FIELDS SEARCHED (Int. Cl.5) C 07 C 271/00 C 07 C 269/00 |

The present search report has been drawn up for all claims

| Place of search | Date of completion of the search | Examiner |
|---|---|---|
| THE HAGUE | 14-03-1990 | WELLS A.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................

& : member of the same patent family, corresponding document

EPO FORM 1503 03.82 (P0401)